# EUROPEAN PATENT SPECIFICATION

(11) **EP 0 607 442 B1**
(45) Date of publication and mention of the grant of the patent: **28.04.1999**
(21) Application number: 93906344.2
(22) Date of filing: 08.10.1992
(51) Int. Cl.: G01N 35/10, G01N 1/28

(54) **METHOD OF DILUTING HIGHLY VISCOUS LIQUID**
METHODE ZUM VERDÜNNEN EINER HOCHVISKOSEN FLÜSSIGKEIT
PROCEDE DE DILUTION DE LIQUIDE HAUTEMENT VISQUEUX

(30) Priority: 08.10.1991 JP 260707/91
(43) Date of publication of application: 27.07.1994
(73) Proprietor: ALOKA CO., LTD., Mitaka-shi Tokyo 181 (JP); ABBOTT LABORATORIES, Abbott Park, Illinois 60064-3500 (US)
(72) Inventor: KAWANABE, Junichi Aloka Co., Ltd., Mitaka-shi Tokyo 181 (JP); TAKEDA, Masaaki Aloka Co., Ltd., Mitaka-shi Tokyo 181 (JP); KATAGI, Hitomi Aloka Co., Ltd. 22-1, Mure 6-chome, Tokyo 181 (JP); KATO, Yuko Aloka Co., Ltd. 22-1, Mure 6-chome, Tokyo 181 (JP); BIELARCZYK, Gregory, A., Lombard, IL 60148 (US); MAGEE, Rosie, L., Calumet City IL 60409-1726 (US)
(74) Representative: Grünecker, Kinkeldey, Stockmair & Schwanhäusser Anwaltssozietät
(86) International application number: JP9201305
(87) International publication number: WO9307495

(56) References cited:
- EP-A- 0 311 588
- JP-A-57 161 552
- JP-A-58 085 168
- JP-A-58 196 461
- JP-A-63 061 954
- JP-B-63 066 468
- US-A- 3 406 635

## Description

### [Detailed Description of the Invention]

### [Industrial Field of the Invention]

The present invention relates to a method for diluting a high viscosity liquid, and more particularly to a method for diluting a red blood cell component of a blood sample, which is employed in an apparatus for pipetting a blood sample.

### [Prior Art]

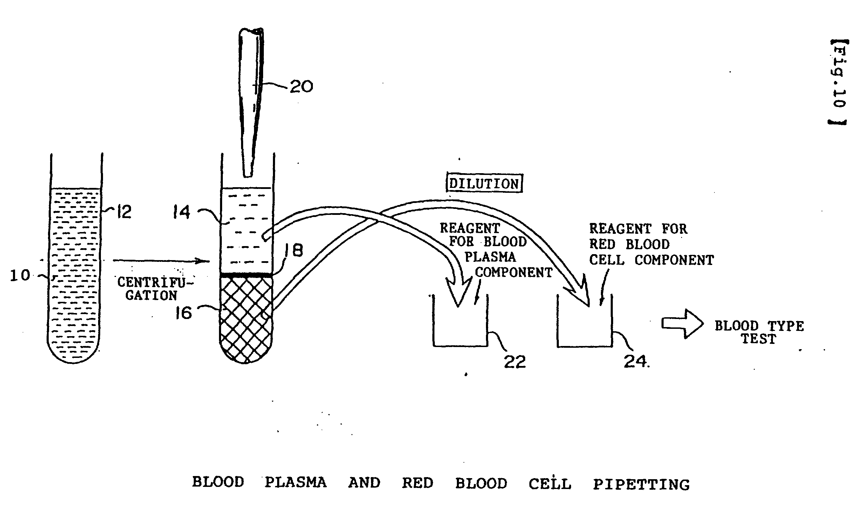

Various kinds of tests are conducted on a blood sample collected from a human body. For example, in a blood type test, as shown in Fig.10, a collected blood sample 10 is put into a test tube 12 and is then separated into a blood plasma component 14 and a red blood cell component 16 by centrifuging it or leaving it as it is. Practically, a small quantity of white blood cell component 18 appears between the blood plasma component 14 and the red blood cell component 16. Since the white blood cell component 18 is not relevant to the following description of the present invention, it is not shown in other drawings.

A blood sample pipetting method which is carried out in a conventional pipetting apparatus comprises two processes including a process of pipetting blood generally plasma and a process of pipetting red blood cells. In the blood plasma pipetting process, the blood plasma component 14 is aspirated through a nozzle tip 20, and then dispensed into a plurality of other recipient containers 22 in a predetermined volume, respectively. In the red blood cell pipetting process, the red blood cell component 16 is aspirated through the nozzle tip 20, and then transferred to a diluting container (not shown) to be mixed with a diluent. Thereafter, a diluted solution of red blood cell component 16 is aspirated again through the nozzle tip 20 and then dispensed into a plurality of other recipient containers 24, respectively in a predetermined volume.

Blood type testing reagents (i.e., a reagent for the blood plasma component and a reagent for the red blood cell component) are introduced into the recipient containers 22, 24, respectively.

Then, these recipient containers 22, 24 are conveyed to an agglutination testing apparatus, where agglutination of the samples in the containers 22, 24 are measured optically or visually. On the basis of the results of the measurements, A type, B type, 0 type or AB type, or Rh type, or the like is determined.

Conventionally, dilution of the red blood cell component has been carried out in the following manner. After having been aspirated by the nozzle tip, the red blood cell component is first dispensed from the nozzle tip into the diluting container. Before or after the dispensation of the red blood cell component, a predetermined volume of diluent is introduced into the diluting container. The red blood cell component is mixed and stirred with the diluent by blowing air into the container or vibrating it. Thereafter, the diluted solution of the red blood cell component is aspirated again through the nozzle tip, and then dispensed into the container 24.

From JP-A-63-61954, there is known a method for preparing sample liquid for analysis comprising discharging a stated amount of a diluent into a prescribed number of wells of each line of a microplate by a nozzle connected with a pump for suction and discharge. A stated amount of a sample is discharged into the first well of each line and thereafter, mixing of the diluent and the sample is executed by repeating several times the suction and discharge. Thereafter, the sample is diluted by absorbing a stated amount of the mixture and discharging it into a second well and mixing it in the same way, whereupon a dilution system is completed by executing the dispensing, mixing and dilution in order for each well in the same way as above.

From JP-A-57-161552, there is known a system in which liquid is aspirated from one container and delivered to another container, followed by delivery thereto of other liquids for mixing or dilution purposes. From EP-A-0311588, there is known a system in which a sample is sucked up to a valve and diluted by a reactive medium directed to the valve. Thereafter, the sample is delivered to a container with further dilution steps performed thereafter. From JP-A-63066468, there is known a system in which several different liquids are sucked up successively into a nozzle.

However, as well known, the red blood cell component is a high viscosity liquid (or a gel-like substance), so it cannot be easily dispensed from the nozzle tip due to its high viscosity. Consequently, quick dilution of the red blood cell component cannot be achieved by the conventional diluting method.

In more detail, since the red blood cell component cannot be smoothly dispensed from a smaller orifice in a distal end of the nozzle tip into the diluting container due to its high viscosity, it takes a relatively long time to dispense the red blood cell component into the diluting container. Consequently, in an apparatus for pipetting a large volume of blood sample, it has been impossible to improve the pipetting ability.

For the above problems, it can be considered to enlarge the size of the smaller orifice in the distal end of the nozzle tip. However, in that event, pipetting precision will be lowered, and there will be caused an inconvenience of leakage or the like, when transferring the liquid sample by using the nozzle tip. Consequently, a diluting method for solving the above problems has been desired especially for a pipetting apparatus, in which nozzle tips having the same shape are used to pipette the blood plasma component and the red blood cell component.

This invention has been made in view of above problems.

It is therefore an object of the present invention to provide a method for diluting as quickly as possible a high viscosity liquid sample, such as the red blood cell component or the like.

The above object is achieved by the subject matter of claim 1 which specifies the inventive method for diluting a high viscosity liquid. Furthermore, the above object is achieved by the subject matter of claim 3, specifying the inventive method for diluting blood for use in a pipetting apparatus including a nozzle tip.

### [Operation of the Invention]

According to the aforementioned features of the present invention, the diluent is first aspirated prior to dispensation of the high viscosity liquid sample from the nozzle tip. After the liquid sample has been diluted to a certain degree, the mixed liquid in the nozzle tip is dispensed. Further dispensation and aspiration are repeated. Thus, it becomes possible to dilute the high viscosity liquid quickly.

Even if the nozzle tip contains the high viscosity liquid sample, it is easy to aspirate and introduce the diluent into the high viscosity liquid sample in the nozzle tip, as compared with the dispensation of the liquid sample. The dispensation of the red blood cell component diluted to some degree with the diluent can be performed more smoothly than dispensation of the pure red blood cell component. As a result, the diluting time can be shortened, as compared with the conventional diluting method. In the present invention, the first stage of the dilution takes place within the nozzle tip.

It is preferable to increase the speed of the dispensing and aspirating operations gradually in the mixing process in accordance with the degree of the dilution.

### [Embodiments]

Embodiments of the present invention will now be described with reference to the accompanying drawings.

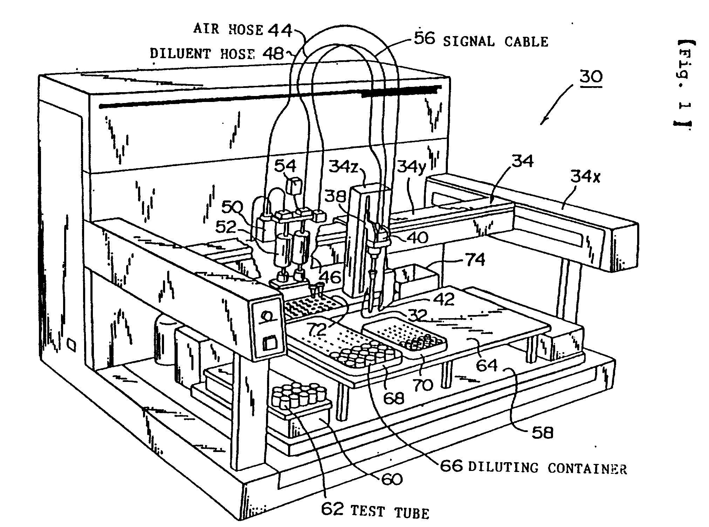

Fig. 1 shows schematically a blood sample pipetting apparatus 30 (hereinafter called "apparatus"), in which a high viscosity liquid diluting method of the present invention is used. Fig. 1 is a perspective view of the apparatus 30.

In this embodiment, the apparatus 30 pipettes the blood plasma component and the red blood cell component after centrifugation to perform a preprocess for blood type test.

As shown substantially in the central portion of Fig. 1, a nozzle 32 for aspirating a blood sample is held by an XYZ robot 34 so as to be movable three-dimensionally.

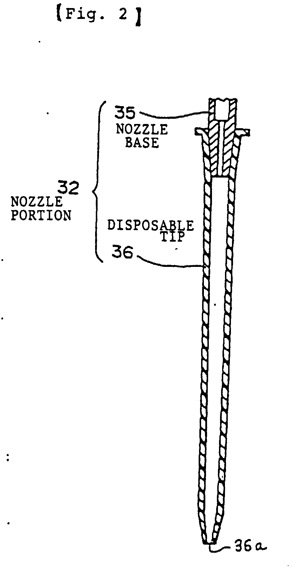

Fig. 2 shows a cross sectional view of a main part of the nozzle 32. The nozzle 32 is composed of a nozzle base 35, and a disposable tip serving as a nozzle tip 36. Thus, the pipetting apparatus in the embodiment of the present invention uses a disposable type nozzle tip. A distal end of the nozzle base 35 is forced into an upper opening of the nozzle tip 36 and is fitted therein. Thus, the nozzle tip 36 is fixed firmly to the nozzle base 35. The nozzle tip 36 has at its lower end a smaller orifice 36a from which the blood sample is aspirated and dispensed. The nozzle tip 36 may be made of a hard plastic material or the like, and the nozzle base 35 may be made of a metal.

In Fig. 1, the XYZ robot 34 is composed of an X drive portion 34x, an Y drive portion 34y and a Z drive portion 34z. To the Z drive portion 34z, an elevator 38 equipped with the nozzle 32 is connected so as to be vertically movable. The elevator 38 has a limit switch 40 serving as a jamming sensor or the like.

The limit switch 40 detects an external force imparted upwardly to the nozzle 32 and having a value greater than a predetermined force.

Onto the Z drive portion 34z, a diluent pipette 42 for dispensing a diluent is fixedly mounted. An air hose 44 is connected at one end thereof to the nozzle 32 and at the other end thereof to a syringe 46 serving as a pump for causing aspirating and dispensing actions. A diluent hose 48 is connected at one end thereof to the diluent pipette 42 and at the other end thereof to a syringe 52 via an electromagnetic valve 50.

Between the syringe 46 and the nozzle 32, a pressure sensor 54 for measuring an internal pressure of the air hose 44 is connected. A signal from the limit switch 40 is sent to the apparatus via a cable 56.

On a test tube rack 60 placed on a pipetting table 58, a plurality of test tubes 62 containing blood samples which have been already subjected to a centrifugation treatment are held uprightly. Each test tube 62, as shown in Fig. 10, contains the blood sample in which the blood plasma component and the red blood cell component are separated in an upper portion and a lower portion of the test tube 62, respectively. On a horizontal table 64 mounted on the pipetting table 58, a dilution tray 68 provided with a plurality of diluting containers 66, and a microplate 70 are provided. On the microplate 70, a plurality of wells are provided for serving as a recipient container for containing the blood plasma component or diluted solution of the red blood cell component. After all of the blood samples have been pipetted, the microplate 70 is conveyed to an apparatus of blood type test, by which an agglutination test, for example, is made optically. The agglutination test may be made visually.

In the apparatus of the present invention, the nozzle tip is a disposable type and is exchanged successively with a new one. A plurality of new nozzle tips are prepared on a nozzle tip stand 72. There is also provided a nozzle scrap tray 74.

Therefore, in the apparatus of the present invention, it is possible that the blood plasma-component or the red blood cell component is aspirated by the nozzle tip 36 of the nozzle 32 and then transferred into other recipient container. The apparatus may also be applied to purposes other than pipetting of the blood sample. Various kinds of applications are possible.

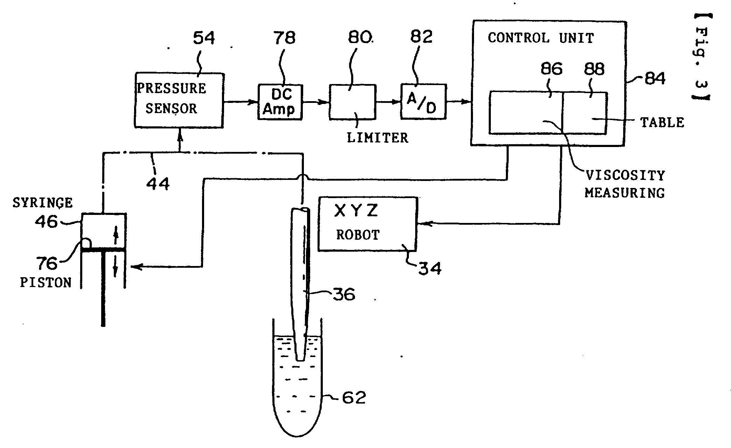

Fig. 3 is a block diagram of the apparatus of the embodiment of the present invention. By moving a piston 76 up and down, inside volume of the syringe 46 varies, so that an aspirating pressure or a dispensing pressure is transmitted to the nozzle tip 36 of the nozzle 32 via the air hose 44 to perform aspiration or dispensation of the blood sample. The internal pressure of the air hose 44 is detected by the pressure sensor 54, a sensor signal outputted from the pressure sensor 54 is amplified by a DC amplifier 78 and is then fed to an analog-digital converter 82 via a limiter circuit 80. The limiter circuit 80 is a protection circuit for suppressing any excessive input. The analog-digital converter 82 converts the sensor signal into a digital signal and feeds the digital signal to a control unit 84.

The control unit 84 includes a computer, for example, for controlling the inside volume of the syringe 46 and the XYZ robot 34, etc. In the embodiment, the control unit 84 also includes a viscosity measuring unit 86 and a table 88, both described below.

Embodiments of a pipetting method of the present invention, used in the above-mentioned apparatus will now be described below.

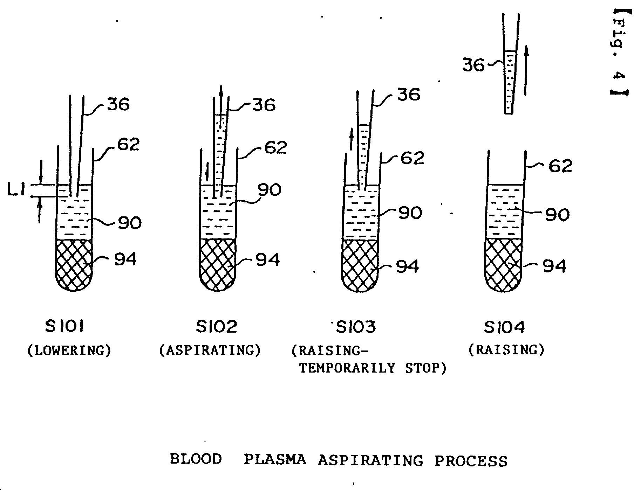

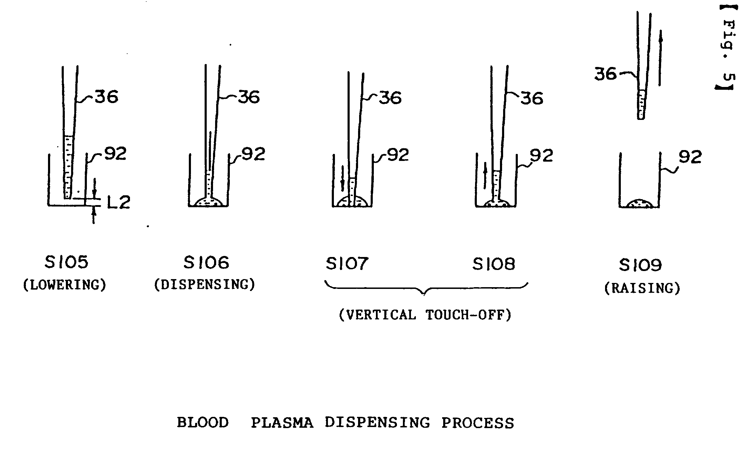

Figs. 4 and 5 show a blood plasma pipetting process. Fig. 4 shows a blood plasma component aspirating process, and Fig. 5 shows a blood plasma component dispensing step.

In Fig. 4, at step 101, the nozzle tip 36 is lowered from the upper side of the test tube 62 and is stopped so that the distal end of the nozzle tip 36 is inserted into the blood plasma component 90 to be positioned at a predetermined distance L1 downward from a blood surface. L1 is preferably 2 to 3 mm. If the distal end of the nozzle tip 36 is inserted too deeply into the blood plasma component 90, the two components once separated by centrifugation are likely to be mixed again with each other.

When the nozzle tip 36 is lowered, the liquid surface is detected. This liquid surface detection is performed by monitoring the internal pressure of the hose 44 by the pressure sensor 54. When the internal pressure of the hose 44 is changed sharply, the control unit 84 detects that the distal end of the nozzle tip 36 has reached the liquid surface.

At step 102, the blood plasma component 90 is aspirated. Specifically, the piston 76 is pulled downwardly to increase the inside volume of the syringe 46, whereby the blood plasma is aspirated into the nozzle tip 36. For example, about 30 to 300 µl of the blood plasma 90 is aspirated. As described in detail below, this aspirated blood plasma component includes a small volume of blood plasma component to be used for a plasma coating. Preferably, the volume of blood plasma component to be aspirated should be determined by taking account of the volume of blood plasma component that finally would attach onto an inner surface of the nozzle tip 36 and would not be dispensed from the nozzle tip 36.

At step 103, the nozzle tip 36 is raised and stopped temporarily just before the distal end of the nozzle tip 36 leaves from the liquid surface of the blood plasma component 90. After a lapse of, for example, about 0. 25 sec., the nozzle tip 36 is raised again at step 104. The reason why the step 103 is needed in this process is that the blood plasma component attaching onto an outer surface of the nozzle tip 36 is returned into the test tube 62, thereby improving the precision of the pipetting. Next to step 104, step 105 of Fig. 5 is executed.

In the blood plasma dispensing process shown in Fig. 5, at step 105, the nozzle tip 36 is lowered into a predetermined well 92 and is stopped so that the distal end of the nozzle tip 36 is positioned at a distance L2 off the bottom of the well 92. In this case, L2 is preferably about 2 mm. If the blood plasma component is dispensed from a too high position, it would become a drop, so that it is difficult to transfer the blood plasma component into the well 92 quickly. Further, if the distal end of the nozzle tip 36 comes into contact with the bottom of the well 92, it becomes very difficult to dispense the blood plasma component.

For these reasons as stated in the above, preferable distance L2 is about 2mm in view of the property of the liquid to be dispensed.

At step 106, a part (predetermined volume) of the blood plasma component existing in the nozzle tip 36 is dispensed.

At step 107, the nozzle tip 36 is lowered until its distal end comes into slight contact with the bottom of the well 92. The contact condition can be monitored by the limit switch 40. At step 108, the nozzle tip 36 is raised. Namely, in step 107 and step 108, a so-called vertical touch-off system is employed, so that the blood plasma component attaching onto the inner surface of the nozzle tip 36 can be dispensed quickly.

At step 109, the nozzle tip 36 is raised and the same dispensing steps (S105-S109) are repeated with respect to other wells. Finally, a predetermined volume of blood plasma component is left in the nozzle tip 36, as shown at step 109 in Fig. 5. This predetermined volume is preferably 15 to 20 µl. The final step 109 of the blood plasma dispensing process shown in Fig. 5 corresponds to a coating preparing step, in which a small volume of blood plasma component is left in the nozzle tip 36 for the purpose of plasma coating.

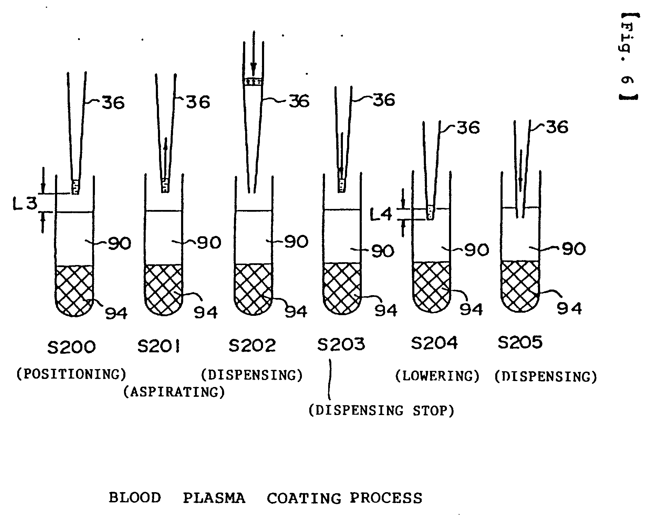

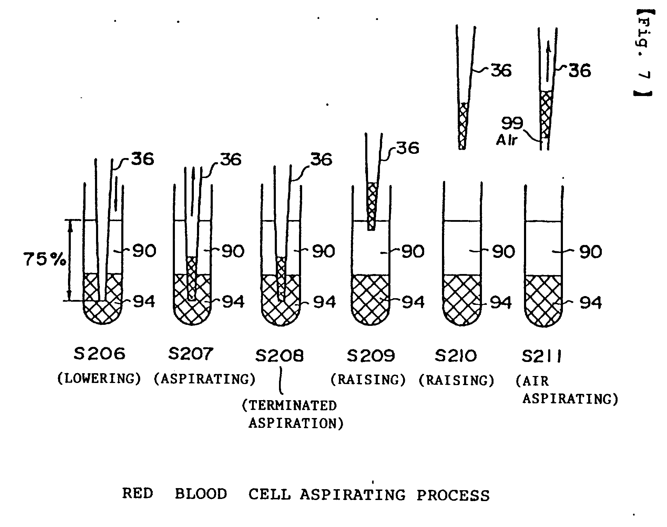

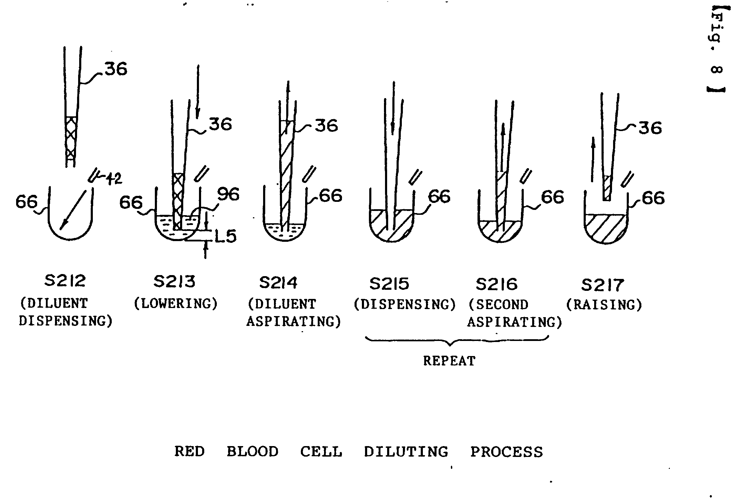

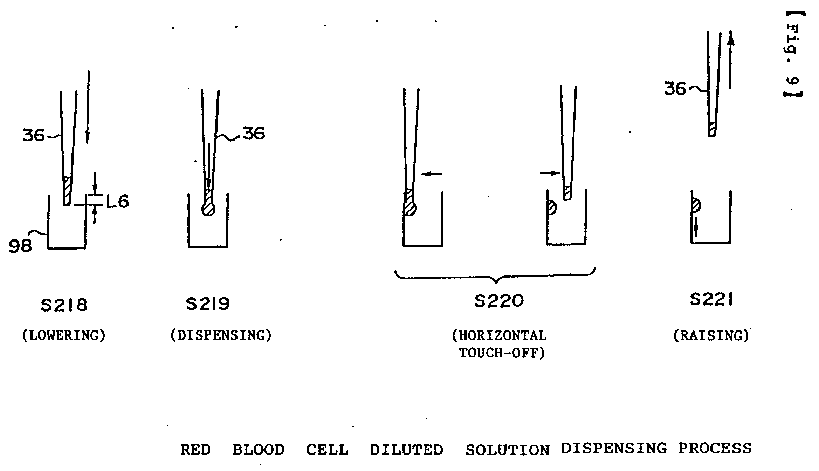

Figs. 6 through 9 show the red blood cell pipetting process, which generally comprises the following four processes including a blood plasma coating process of Fig. 6; a red blood cell aspirating process of Fig. 7; a red blood cell diluting process of Fig. 8; and a red blood cell diluted solution dispensing process of Fig. 9.

Firstly, the blood plasma coating process will now be described with reference to Fig. 6. After step 109 of Fig. 5, step 200 of Fig. 6 is executed. Namely, by the XYZ robot 34, the nozzle tip 36 is positioned above the test tube 62 from which the blood plasma component has been aspirated at step 102. Specifically, the nozzle tip 36 is positioned so that its distal end is inserted slightly (to be positioned at a distance L3 off the blood surface) into the upper opening of the test tube 62. This positioning would prevent the coating blood plasma from scattering over other blood samples and contaminating them, thus improving the reliability of the apparatus. L3 at step 200 is preferably 5 mm.

In the method of this embodiment, pipetting operation is performed continuously by using the same nozzle tip 36 throughout the blood plasma pipetting process and the red blood cell pipetting process. Alternatively, when these two steps processes changeover, the nozzle tip 36 may be exchanged with a new one. In such case, it is necessary to provide a blood plasma component coating step as a preprocessing step before step 200.

At step 201, the blood plasma component used for the coating which remains in the nozzle tip 36 is aspirated (raised) again at least to such an extent that the red blood cell component to be subsequently aspirated reaches. Strictly, the inner surface of tip 36 is coated to such an extent that the red blood cell component diluted later, i.e., the diluted solution of red blood cells reaches.

At step 202, the blood plasma component for the coating is dispensed (lowered), and the dispensation is stopped when the plasma component reaches the distal end of the nozzle tip 36 as shown at step 203 of Fig. 6.

In this embodiment, the coating is accomplished by moving the blood plasma component up and down only one time as shown at step 201 and step 202. Alternatively, the blood plasma component may be moved up and down two or more times, if necessary. However, it is confirmed that moving up and down one time is sufficient for the coating.

At step 204, the nozzle tip 36 is lowered and stopped when its distal end is inserted into the blood plasma component 90 to be positioned at a distance L4 downward from the blood surface. L4 is preferably 2 to 3 mm. At step 205, the remaining blood plasma component used for the coating is returned into the test tube 62. Thus, in order to use a valuable blood sample without wasting it, the blood plasma component for coating is returned into the test tube 62. Although only a small volume of blood plasma component is used for the coating in this embodiment, a large volume of blood plasma component may be used for the coating. If a large volume of blood plasma component is used, it is necessary to aspirate an unnecessarily large volume of blood plasma component at step 102, and at step 205, when returning such large volume of blood plasma component into the tube, there is a risk that the blood plasma component is mixed with the red blood cell component. Consequently, it is preferable that the coating should be made with a small volume of blood plasma component.

After step 205, step 206 of Fig. 7 is executed. At step 206, the nozzle tip 36 is further lowered, so that its distal end is inserted into the red blood cell component 94. At that time, it is preferable that the distal end of the nozzle tip 36 is located at a position of about 75% downward from the surface of the blood plasma component, where the whole blood sample is 100%. If the nozzle tip 36 is inserted into the blood plasma component too deeply, a large volume of red blood cell component 94 attaches onto the outer surface of the nozzle tip 36. In contrast, if the nozzle tip 36 is inserted into the blood plasma component too shallowly, it is difficult to aspirate the red blood cell component surely.

At step 207, the red blood cell component 94 is aspirated. In this embodiment, for example, 80 µl of red blood cell component 94 is aspirated.

However, the red blood cell component 94 is a high viscosity liquid or a gel substance, so that considerable pressure and time are needed to aspirate such red blood cell component via the smaller orifice of the distal end of the nozzle tip 36. In this embodiment, the following two ways are used to improve the quickness of aspiration to a maximum extent. In the first way, the inner surface of the nozzle tip 36 is coated with the blood plasma component in order to reduce the frictional resistance of the inner surface of the nozzle tip 36 as much as possible, thus ensuring a smooth aspiration of the red blood cell component. In the second way, an excessively large volume of red blood cell component is aspirated temporarily. Specifically, the piston 76 of the syringe 46 is drawn to a maximum extent, and thereafter the internal pressure of the air hose 44 is monitored by the pressure sensor 54. When the internal pressure becomes equal to a predetermined value, the piston 76 is returned, so that only a desired volume of red blood cell component is finally aspirated into the nozzle tip 36. That is, since a high viscosity solution of red blood cell component cannot be aspirated so as to quickly follow the moving amount of the syringe 46, the maximum aspiration force is applied to the nozzle 36, at an initial aspiration stage, the moving amount of the syringe 46 is returned to a proper value just before a desired volume of red blood cell component is aspirated, thereby obtaining a predetermined volume of aspirated red blood cell component of which volume is to be determined by the moving amount of the syringe 46. At step 208, when a detection value of the pressure sensor 54 becomes substantially equal to an atmospheric pressure, the termination of aspiration is confirmed. Strictly, since the red blood cell component is aspirated into the nozzle tip 36, the internal pressure of the air hose 44 has a tendency to become lower than the atmospheric pressure. Finally, at step 208, for example, 80 µl of the red blood cell component is aspirated into the nozzle tip 36.

At step 209, the nozzle tip 36 is raised slowly in order to keep the two blood components stable in a separated state and in order to avoid causing any turbulent flow. Near the liquid surface of the blood plasma component 90, the nozzle tip 36 is temporarily stopped from raising as shown in step 209, and thereafter the nozzle tip 36 is raised again at step 210.

At step 211, for example, about 10 µl of air is aspirated to provide an air cap 99 at the distal end of the nozzle tip 36. This enables to completely take in the nozzle tip 36 the red blood cell component attaching to an edge of the distal end of the nozzle tip 36, and to prevent the red blood cell component from dropping from the nozzle tip 36.

The diluting process according to the present invention will now be described.

After step 211, step 212 of Fig. 8 is executed. In the step 212, the nozzle tip 36 is conveyed to an upper position above the diluting container 66, at which a predetermined volume of diluent is dispensed into the diluting container 66 through the diluent pipette 42. At step 213, the nozzle tip 36 is lowered from the upper position, so that its distal end is positioned in the diluent 96 at a predetermined distance L5 off the bottom of the diluting container 66. L5 is preferably 2 mm. A physiological salt solution can be used for the diluent.

At step 214, the diluent 96 is aspirated into the nozzle tip 36. In the conventional method, the red blood cell component was dispensed to be mixed and diluted with the diluent. However, in this embodiment, in view of the facts that the red blood cell component is a high viscosity and dispensation thereof is relatively difficult, the diluent which is easy to be aspirated is previously aspirated, and then the diluent is mixed with the red blood cell component step by step.

At step 215, the mixed solution is dispensed from the nozzle tip 36 into the diluting container 66. At that time, since the red blood cell component is previously diluted by the diluent to a certain degree, the mixed solution can be dispensed extremely easily, as compared with the dispensation of a pure red blood cell component. At step 216, the mixed solution, i.e., the diluted solution of red blood cell is aspirated. In this embodiment, step 215 and step 216 are repeated about five times. During the successive steps from step 214, the aspiration or dispensation is initially performed slowly, depending on the viscosity of the substance to be aspirated or dispensed, and thereafter progressively more quickly. At step 214, since the inner surface of the nozzle tip 36 has been previously coated with the blood plasma component, the diluent can be aspirated into the nozzle tip 36 more smoothly than the case in which the inner surface of the nozzle tip 36 is not coated with the blood plasma component.

At step 217, the nozzle tip 36 is stopped temporarily from being raised when its distal end reaches near the liquid surface, and then the nozzle tip 36 is raised again.

A process of dispensing the diluted solution of the red blood cell solution will now be described.

After step 217, step 218 of Fig. 9 is executed. The nozzle tip 36 is conveyed to a predetermined well 98 on the microplate by the XYZ robot. The nozzle tip 36 is lowered, so that its distal end is positioned in the well 98 at a predetermined distance L6 downward from the upper opening of the diluting container 66, as shown at step 218. L6 is preferably about 3 mm. Though there is not shown in the drawings, a predetermined volume of reagent has been previously pipetted into the respective wells 98.

At step 219, a predetermined volume of diluted solution of red blood cell component in the nozzle tip 36 is dispensed, and then a drop of the diluted solution formed at the distal end of the nozzle tip 36 is attached onto the inner surface of the well 98 at step 220. Namely at step 220, by using the horizontal touch-off system, the diluted solution of red blood cell component is dispensed, without bringing the distal end of the nozzle tip 36 into direct contact with the reagent in the lower portion of the well, thereby preventing contamination from being caused.

At step 221, the nozzle tip 36 is raised and then moved to a next well, and thereafter the steps from step 218 to step 221 are repeatedly executed.

In this embodiment, after the blood plasma pipetting process and the red blood cell pipetting process have been executed for a specific blood sample, the nozzle tip 36 is exchanged with a new one. By using this new nozzle tip, the blood plasma pipetting process and the red blood cell pipetting process are executed in the same way for other blood samples.

The viscosity measuring unit 86 of Fig. 3 will now be described hereinbelow. The viscosity of the red blood cell component is valuable information for diagnosing an illness and is also a valuable material for determining an aspiration pressure to be set when a blood sample is pipetted. In the conventional apparatus, there is not provided any viscosity measuring unit, and the viscosity of a blood sample is measured by a separate measuring instrument. Therefore, after having been subjected to the viscosity measurement, the blood sample has been disused. Further, the viscosity measurement takes a long time and needs a complex operation.

In the apparatus of the present invention, since the control unit 84 is equipped with the viscosity measuring unit 86, it is possible to measure the viscosity of the blood sample while the blood sample being pipetted.

In various liquids having different viscosities, there is a close relationship between the viscosity of each liquid and the time required from a point of time when a predetermined pressure is produced as an initial aspiration pressure for aspirating the liquid to a point of time when the aspiration pressure reaches a certain pressure value. It was confirmed in a simulation that there is a proportional relation ship therebetween.

In a table 88, there is stored data which represents a relationship between the viscosity of the liquid to be aspirated and the time required from a point of time when the liquid begins to be aspirated under a certain initial aspiration pressure to a point of time when the pressure which returns gradually to an atmospheric pressure reaches a specifically set. pressure value.

Specifically, when aspirating the blood sample into the nozzle tip 36, the internal pressure of the air tube 44 is monitored by the pressure sensor 54, and the viscosity measuring unit 86 measures the time required from a point of time when the piston 76 is drawn to give the initial aspiration pressure, to a point of time when the internal pressure of the air tube 44 reaches a predetermined value. The viscosity measuring unit 86 also obtains a viscosity of the blood sample by comparing the measured time with the table 88. The obtained viscosity is indicated on a display unit (not shown), and this result is utilized for controlling the syringe 46 by the control unit 84.

According to the viscosity measuring unit 86 described above, there are advantages as follows. Since the viscosity can be measured simultaneously with the aspiration of the blood sample, the unit 86 requires no additional time for measuring the viscosity, and realizes a very simple viscosity measurement. Further, it is unnecessary to prepare an additional blood sample only for viscosity measurement.

### [Advantageous Effect of the Invention]

As explained in the above, in the mixing process of the present invention, the diluent is aspirated before the red blood cell component is dispensed from the nozzle tip into the diluting container. Thus, the dilution at a first stage is performed by using the nozzle tip. Then, the diluted red blood cell component is repeatedly transferred between the nozzle tip and the diluting container to perform dilution and mixing. As a result, the method of the present invention makes it possible to dilute a high viscosity liquid quickly, as compared with.the conventional diluting method.

Therefore, the present invention has an advantage of improving the pipetting ability when applied to an apparatus for pipetting a red blood cell component or the like.

### [Brief Explanation of the Drawings]

Fig. 1 is a schematic view for showing an embodiment of a pipetting apparatus in which a diluting method of the present invention is used;
Fig. 2 is a cross-sectional view showing a main part of a nozzle;
Fig. 3 is a block diagram of the pipetting apparatus of Fig. 1;
Fig. 4 is an explanatory view for showing steps, in which blood plasma is aspirated into the nozzle during a process of pipetting blood plasma;
Fig. 5 is an explanatory view for showing steps, in which the blood plasma is dispensed from the nozzle during a process of pipetting the blood plasma;
Fig. 6 is an explanatory view for showing steps, in which the inner surface of the nozzle is coated with the blood plasma during a process of pipetting red blood cells;
Fig. 7 is an explanatory view for showing steps, in which red blood cells are aspirated into the nozzle during the process of pipetting the red blood cells;
Fig. 8 is an explanatory view for showing steps, in which the red blood cells are diluted during the process of pipetting the red blood cells;
Fig. 9 is an explanatory view for showing steps, in which a red blood cell diluent is dispensed from the nozzle during the process of pipetting the red blood cells; and
Fig. 10 is an explanatory view for showing an operation of pipetting the blood plasma and the red blood cells as a preprocess for a blood type test.

### [Explanation of References Numerals]

- 30: pipetting apparatus
- 32: nozzle
- 34: XYZ robot
- 35: nozzle base
- 36: disposable tip
- 54: pressure sensor
- 84: control unit
- 86: viscosity measuring unit
- 90: blood plasma component
- 91: red blood cell component

## Claims

1. A method for diluting a high viscosity liquid such as blood and the like for use in a pipetting apparatus, the method comprising the steps of:
(a) aspirating the high viscosity liquid into a nozzle tip of the pipetting apparatus through a lower end of the nozzle tip;
(b) inserting the lower end of the nozzle tip containing the high viscosity liquid into a diluent container containing a diluent and maintaining the lower end of the nozzle tip in the diluent container during performance of steps (c), (d) (e) and (f);
(c) aspirating the diluent into the nozzle tip to dilute the high viscosity liquid contained in the nozzle tip with the aspirated diluent forming diluted high viscosity liquid;
(d) dispensing the diluted high viscosity liquid into the diluent container;
(e) aspirating the diluted high viscosity liquid from the diluent container, and
(f) repeating dispensing step (d) and aspirating step (e).

2. A method as defined in claim 1 wherein speed of the dispensing step (d) and the aspirating step (e) are gradually increased.

3. A method for diluting blood for use in a pipetting apparatus including a nozzle tip, the method comprising the steps of:
(a) positioning the nozzle tip in a container containing blood separated vertically into a blood plasma component and a red blood cell component;
(b) aspirating the red blood cell component into the nozzle tip through a lower end of the nozzle tip;
(c) inserting the lower end of the nozzle tip containing the red blood cell component into a diluent container containing a diluent and maintaining the lower end of the nozzle tip in the diluent container during performance of steps (d), (e), (f), and (g)
(d) aspirating the diluent into the nozzle tip to dilute the red blood cell component contained in the nozzle tip with the aspirated diluent forming diluted red blood cell component;
(e) dispensing the diluted red blood cell component into the diluent container;
(f) aspirating the diluted red blood cell component from the diluent container, and
(g) repeating dispensing step (e) and aspirating step (f).

4. A method as defined in claim 3 wherein speed of the dispensing step (e) and the aspirating step (f) are gradually increased.

## Patentansprüche

1. Verfahren zum Verdünnen einer hochviskosen Flüssigkeit, wie beispielsweise Blut und dergleichen, zur Verwendung in einer Pipettiervorrichtung, wobei das Verfahren die Schritte aufweist:
(a) Absaugen der hochviskosen Flüssigkeit in eine Düsenspitze der Pipettiervorrichtung hinein über ein unteres Ende der Düsenspitze;
(b) Einsetzen des unteren Endes der Düsenspitze, die die hochviskose Flüssigkeit enthält, in einen Verdünnungsmittelbehälter hinein, der ein Verdünnungsmittel enthält, und Beibehalten des unteren Endes der Düsenspitze in dem Verdünnungsmittelbehälter während der Durchführung der Schritte (c), (d), (e) und (f);
(c) Absaugen des Verdünnungsmittels in die Düsenspitze hinein, um die hochviskose Flüssigkeit, die in der Düsenspitze enthalten ist, mit dem abgesaugten Verdünnungsmittel zu verdünnen, was eine verdünnte, hochviskose Flüssigkeit bildet;
(d) Abgeben der verdünnten, hochviskosen Flüssigkeit in den Verdünnungsmittelbehälter hinein;
(e) Absaugen der verdünnten, hochviskosen Flüssigkeit von dem Verdünnungsmittelbehälter, und
(f) Wiederholen des Abgabeschritts (d) und des Absaugschritts (e).

2. Verfahren nach Anspruch 1, wobei die Geschwindigkeit des Abgabeschritts (d) und des Absaugschritts (e) graduell erhöht wird.

3. Verfahren zum Verdünnen von Blut zur Verwendung in einer Pipettiervorrichtung, die eine Düsenspitze umfaßt, wobei das Verfahren die Schritte aufweist:
(a) Positionieren der Düsenspitze in einem Behälter, der Blut enthält, das vertikal in eine Komponente aus Blutplasma und in eine Komponente aus roten Blutzellen separiert ist;
(b) Absaugen der Komponenten der roten Blutzellen in die Düsenspitze hinein über ein unteres Ende der Düsenspitze;
(c) Einsetzen des unteren Endes der Düsenspitze, die die Komponente der roten Blutzellen enthält, in einen Verdünnungsmittelbehälter hinein, der ein Verdünnungsmittel enthält, und Beibehalten des unteren Endes der Düsenspitze in dem Verdünnungsmittelbehälter während der Durchführung der Schritte (d), (e), (f) und (g);
(d) Absaugen des Verdünnungsmittels in die Düsenspitze hinein, um die Komponente der roten Blutzellen, die in der Düsenspitze enthalten ist, mit dem abgesaugten Verdünnungsmittel zu verdünnen, was eine verdünnte Komponente aus roten Blutzellen bildet;
(e) Abgeben der verdünnten Komponenten der roten Blutzellen in den Verdünnungsmittelbehälter hinein;
(f) Absaugen der verdünnten Komponenten der roten Blutzellen von dem Verdünnungsmittelbehälter; und
(g) Wiederholen des Abgabeschritts (e) und des Absaugschritts (f).

4. Verfahren nach Anspruch 3, wobei die Geschwindigkeit des Abgabeschritts (e) und des Absaugschritts (f) graduell erhöht wird.

## Revendications

1. Procédé pour diluer un liquide de viscosité élevée tel que du sang et analogue en vue d'une utilisation dans un appareil de pipetage, le procédé comprenant les étapes consistant:
(a) à aspirer le liquide de viscosité élevée dans un embout de buse de l'appareil de pipetage par une extrémité inférieure de l'embout de buse;
(b) à insérer l'extrémité inférieure de l'embout de buse contenant le liquide de viscosité élevée dans un récipient de diluant contenant un diluant et à maintenir l'extrémité inférieure de l'embout de buse dans le récipient de diluant pendant l'exécution des étapes (c), (d), (e) et (f);
(c) à aspirer le diluant dans l'embout de buse afin de diluer le liquide de viscosité élevée contenu dans l'embout de buse avec le diluant aspiré en formant un liquide de viscosité élevée dilué;
(d) à distribuer le liquide de viscosité élevée dilué dans le récipient de diluant;
(e) à aspirer le liquide de viscosité élevée dilué à partir du récipient de diluant, et
(f) à répéter l'étape de distribution (d) et l'étape d'aspiration (e).

2. Procédé selon la revendication 1, dans lequel la vitesse de l'étape de distribution (d) et de l'étape d'aspiration est augmentée progressivement.

3. Procédé pour diluer du sang en vue d'une utilisation dans un appareil de pipetage comprenant un embout de buse, le procédé comprenant les étapes consistant:
(a) à disposer l'embout de buse dans un récipient contenant du sang séparé verticalement en un composant de plasma sanguin et en un composant d'érythrocytes;
(b) à aspirer le composant d'érythrocytes dans l'embout de buse par une extrémité inférieur de l'embout de buse;
(c) à insérer l'extrémité inférieure de l'embout de buse contenant le composant d'érythrocytes dans un récipient de diluant contenant un diluant et à maintenir l'extrémité inférieure de l'embout de buse dans le récipient de diluant pendant l'exécution des étapes (d), (e), (f) et (g);
(d) à aspirer le diluant dans l'embout de buse pour diluer le composant d'érythrocytes sanguin contenu dans la pointe de buse avec le diluant aspiré afin de former le composant d'érythrocytes dilué;
(e) à distribuer le composant d'érythrocytes dilué dans le récipient de diluant;
(f) à aspirer le composant d'érythrocytes dilué à partir du récipient de diluant; et
(g) à répéter l'étape de distribution (e) et l'étape d'aspiration (f).

4. Procédé selon la revendication 3, dans lequel la vitesse de l'étape de distribution (e) et de l'étape d'aspiration (f) est augmentée progressivement.
